(19) **Europäisches Patentamt**

**European Patent Office**

**Office européen des brevets**

(11) Publication number: **0 237 252 B1**

(12) **EUROPEAN PATENT SPECIFICATION**

(45) Date of publication of patent specification: **12.10.94**

(51) Int. Cl.[5]: **G01N 33/574**, A61K 39/385, C12P 21/08, G01N 33/68, G01N 33/543, C07K 3/20, G01N 33/577, C12N 15/00, C07K 15/28

(21) Application number: **87301837.8**

(22) Date of filing: **03.03.87**

(54) **Methods and compositions relating to regression-associated antigens.**

(30) Priority: **07.03.86 US 837494**
**18.02.87 PCT/US87/00334**

(43) Date of publication of application:
**16.09.87 Bulletin 87/38**

(45) Publication of the grant of the patent:
**12.10.94 Bulletin 94/41**

(84) Designated Contracting States:
**AT BE CH DE ES FR GB GR IT LI LU NL SE**

(56) References cited:
**EP-A- 0 092 223**
**WO-A-85/02467**
**US-A- 4 562 160**

**CHEMICAL ABSTRACTS, vol. 80, no. 13, 01 April 1974, Columbus, OH (US); J.S. GREEN-BERGER et al., p. 206, no. 68973b&NUM;**

**BIOLOGICAL ABSTRACTS, vol. 74, no. 10, 1982; C. VENNEGOOR et al.&NUM;**

(73) Proprietor: **Xoma Corporation**
**2910 7th Street**
**Berkeley California 94710 (US)**

(72) Inventor: **Fareed, George C.**
**721 N. Thayer Avenue**
**Los Angeles, CA 90024 (US)**
Inventor: **Sen, Arup**
**3430 Jasmine Avenue,**
**No. 202**
**Los Angeles, CA 90034 (US)**
Inventor: **Ghosh-Dastidar, Pradip**
**3556 S. Barrington Avenue**
**Los Angeles, CA 90066 (US)**
Inventor: **Liu, Alvin Y.**
**807 5th St.,**
**Apt. 6**
**Santa Monica, CA 90403 (US)**

(74) Representative: **Brown, John David**
**FORRESTER & BOEHMERT**
**Franz-Joseph-Strasse 38**
**D-80801 München (DE)**

CANCER RESEARCH, vol. 37, August 1977, Chicago, IL (US); O.P. VAN DIGGELEN et al., pp. 2680-2687&NUM;

PROCEEDINGS OF THE NATL. ACADEMY OF SCIENCES USA, vol. 83, May 1986, Washington, DC (US); P.K. SRIVASTAVA et al., pp. 3407-3411&NUM;

CHEMICAL ABSTRACTS, vol. 105, no. 25, 25 December 1986, Columbus, OH (US); H.J. STRAUSS et al., p. 583, no. 224139b&NUM;

## Description

Background

The present invention pertains in general to antigens associated with tumors and uses therefor, and in particular to regression-associated antigens (RAAs), to preparations containing RAAs and to uses for such antigens and preparations.

One approach to diagnosis and treatment of cancers involves the development of polyclonal and monoclonal antibodies against tumor-associated antigens. In almost all reported cases, the immunogens used to obtain antibodies directed against tumor cell components are intact tumor cells or are membrane proteins obtained from the cells.

Early work in this field involved the identification of onco-fetal antigens and blood group antigens [Springer, Science, 224, 1198 (1984)] which are expressed by malignant cells and shed into the bloodstream in some instances. Antigens associated with tumor cells may be identified by immunoblotting methods. Du Bois et al., J. Immunol. Methods, 63, 7 (1983).

In particular, monoclonal antibodies reactive with the surface of human breast carcinoma cells may be generated and characterized using membrane-enriched fractions of metastatic carcinoma lesions. Schlom et al., Cancer, 54 (11 suppl.), 2777-2794 (1984). One monoclonal antibody reacts with a 220,000 to 400,000 dalton high molecular weight glycoprotein complex found in 50% of human mammary carcinomas and 80% of human colon carcinomas. Scholm et al., supra. Mouse monoclonal antibody L6 recognizes a ganglioside antigen that is of particular interest because it is expressed at the surface of cells from most human carcinomas of lung, breast, colon and ovary, while it is present in only trace amounts at the surface of normal cells. Hellstrom et al., Cancer Res., 46, 3917-3923 (1986).

A number of other monoclonal antibodies reactive with tumor-associated antigens on the surfaces of other human cancer cells, including ovarian, pancreatic and intestinal malignancies, may thus be obtained. See the Proceedings of the UCLA Symposium on Monoclonal Antibodies and Cancer Therapy, Reisfeld et al., eds., Alan R. Liss, Inc., New York, pp. 3-74, 97-109 and 149-164 (1985).

Monoclonal and polyclonal anti-tumor cell antibodies described to date are directed against determinants of human tumor cell antigens which may elicit an immune response in test animals chosen for the production of tumor-specific antibodies. It is not known whether patients harboring tumors or treated with specific and/or non-specific immune stimulants produce antibodies against these antigenic determinants. Therefore, the relevance of such antibodies in mediating regression of tumors in patients is unclear. Passive transfer of such antibodies generated in animals into patients has met with limited success. Lowder et al., Western J. Med., 143, 810 (1985).

A clinical approach toward active immunotherapy of tumors involves a generalized stimulation of the patient's own immune system using non-specific stimulants such as components of the walls of two bacterial cells, Mycobacterium bovis (BCG strain) and Corynebacterium parvum (C. parvum), or "detoxified" bacterial endotoxin. In parallel, biological response modifiers such as interleukin-2 may be used to induce activation of the immune system and cause tumor cell destruction. Mule et al., J. Immunol., 135, 646 (1985); and Rosenberg et al., New Engl. J. Med., 313, 1485 (1985).

In an approach called active specific immunotherapy, immunization of cancer patients may be attempted with preparations derived from allogeneic tumor cells (tumor cells obtained from a histopathologically similar tumor of a different patient). This may specifically stimulate the patient's own immune system to possibly unique antigenic structures present on a particular malignant cell type, and may thus induce tumor regression. Lachman et al. Br. J. Cancer, 51, 415-417 (1985); and Wallack et al., Surgery, 96, 791-800 (1984). Active specific immunotherapy may also be attempted by systematically injecting autologous (autochthonous) tumor cells (i.e., cells derived from the tumor mass of the same patient) intradermally or subcutaneously. Laucius et al., Cancer, 40, 2091 (1977).

Various preparations of autologous tumor cells or of allogeneic tumor cell lines have been used in active specific immunotherapy. Key et al., Adv. Immun. Cancer Ther., 1, 195-219 (1985); Weisenburger et al., J. Biol. Response Mod., 1, 57-66 (1982); and Kan-Mitchell et al., Proceedings of the UCLA Symposium on Monoclonal Antibodies and Cancer Therapy, supra, 523-536. The preparations are generally treated with irradiation, mechanical disruption, or freeze-thaw cycles to render the tumor cells non-viable. They are then used as immunogens, with or without an adjuvant, and are administered by a variety of routes (such as intradermal, subcutaneous, intramuscular or intralymphatic) for the purpose of immunizing cancer patients. Relatively little toxicity has been reported with these preparations, and encouraging clinical responses have been obtained in significant numbers of advanced cancer patients.

One major limitation of attempts at active specific immunotherapy is the undefined nature of the tumor cell preparations (generally intact irradiated cell suspensions or mechanically disrupted lysates of cells). Cells from autologous tumor cells grown in tissue culture or continuously passaged tumor cell lines may undergo significant changes in their phenotypes during growth in laboratory culture. Reagents or tests to standardize the preparations for their expected potency have not been available. Membrane proteins may be shed from intact irradiated cells and proteins in cell lysates may be degraded by proteolytic enzymes. Different preparations used in attempts at active specific immunotherapy may, therefore, have variable efficacies although similar processes and cell types are utilized. Furthermore, the means of monitoring the immune response of individual patients is not available for tailoring the immunization dose and the immunization schedule for optimal clinical outcome. In addition, it is often the case that autologous tumor cell preparations are not practical because of a lack of adequate tumor from the patient to be treated.

Results from a number of animal models support the use of tumor cell components in active specific immunization to induce tumor regression [Key et al., J. Biol. Response Mod., 3, 359-365 (1984); and Srivastava et al., Proc. Nat'l Acad. Sci. (USA), 83, 3407-3411 (1986)]. Neoplasms induced in mice by polycyclic aromatic hydrocarbons such as 3-methylcholoanthrene express individually distinct tumor-associated transplantation antigens. These antigens are immunogenic in their syngeneic hosts and provide transplantation immunity only against their respective tumors and not against independent tumors induced by the same or a different carcinogen or against tumors of viral origin. Transplantation immunity in mice may be elicited by prior growth and removal of tumor transplants or by immunization with irradiated tumor cells, tumor cell membranes or solubilized antigen preparations.

The results of clinical studies with autologous tumor cell vaccines are encouraging when a potent adjuvant such as BCG is used along with the tumor cell suspension. Hoover et al., Cancer Res., 44, 1671-1676 (1984). Some patients immunized through different routes with allogeneic cells with or without adjuvants have shown significant, often dramatic, clinical responses. Weisenburger, J. Biol. Response Mod., 1, 57-66 (1982); Mitchell, in Proceedings of the UCLA Symposium on Monoclonal Antibodies and Cancer Therapy, supra, 495-504. Certain key antigen components of tumor cells may be able to to elicit protective/regressor antibodies in humans.

A knowledge of the antibody response associated with human tumor regression following active specific immunotherapy and identification of subcellular components involved in eliciting such specific antibodies should lead to the development of improved active specific immunogens for cancer immunotherapy. Thus, it is desirable to develop: (i) preparations which will be more enriched in the relevant specific immunogens; (ii) reagents to screen better cell sources and quantitate the immunogens in preparations derived from these cells such that different preparations can be meaningfully standardized; and (iii) assay methods to monitor patients' specific immune response to these immunogens, thereby providing the physician an ability to adjust the treatment protocol in order to produce a better clinical outcome.

Summary of the Invention

Regression-associated antigens may be identified based upon their reactivity with regression-associated antibodies (RAAbs) which antibodies are produced in patients undergoing active immunization and responding with tumor regression.

A method for detecting RAAbs according to the present invention involves obtaining a first sample of serum from a patient diagnosed as not being in a state of regression and then obtaining a second sample of serum from the patient after diagnosis as being in a state of regression. Protein extracts of a neoplastic cell are exposed to each of the samples. The formation of an immune complex between a component of the neoplastic cell and an antibody in the second serum sample and the absence of such a complex with an antibody in the first serum sample is indicative of the presence of RAAbs in the second sample.

A method for purifying RAAs according to the present invention involves disrupting the neoplastic cell with associated RAAs (as determined by complex formation with an RAAb) into components of a solution, separating the component of the solution into fractions and identifying fractions containing RAAs by immune complex formation with RAAbs. A purified and isolated RAA according to the present invention has a molecular mass as determined by reducing SDS polyacrylamide gel electrophoresis to be within the range from 19000 to 23000 daltons, or within the range from 38000 to 45000 daltons or within the range from 65000 to 71000 daltons. Preferably these regression associated antigens having the above molecular masses do not bind to cationic exchange resins, such as DEAE Sephacel®, at 50 mM $Na_3PO_4$ (pH7) or to heparin agarose resins at 200 mM $Na_3PO_4$ (pH 7). More preferably, wherein the regression associated antigen is greater than or equal to regression associated antigen is greater than or equal to 95% pure as determined by reducing SDS PAGE analysis followed by silver staining. The present invention also

EP 0 237 252 B1

comprehends regression associated antigens encoded by DNA from a mammalian cell line which may have an infection with Mycoplasma hyorhinis and regression associated antigens encoded by DNA of Mycoplasma hyorhinis.

Different materials for immunotherapy may be prepared and immunotherapy may be performed according to the present invention by introducing an RAA according to the present invention, a neoplastic cell containing one or more of the molecular species of RAAs according to the present invention, or a cell-free preparation (e.g. particulate preparation or protein released in the culture fluid) from such a neoplastic cell into the lymphatic or hematic fluid (i.e., the lymph or blood) or into tissues of a patient (e.g. by intramuscular or subcutaneous injection). The response of a patient to immunotherapy may be monitored for symptoms and signs associated with a neoplasm and by determining a circulating level of RAAbs in a patient.

The present invention also provides monoclonal or monospecific polyclonal antibodies exhibiting a specific immunoreactivity with an RAA. An antibody according to the present invention may be purified by contacting a substrate bound RAA with a solution containing an RAAb and eluting the RAAb from the RAA.

A monoclonal or monospecific polyclonal antibody directed against one or more of the RAAs of the present invention may be administered to cause direct antibody-dependent tumor cell cytotoxicity (ADCC) or complement-dependent tumor cell cytotoxicity (CDCC). Alternatively, such an antibody may be bound to bioactive moiety including but not limited to an anticancer drug, such as toxins or chemotherapeutic substances, or cell growth and differentiation regulators (e.g., IL-1,TGF-$\beta$, TNF, IFN and the like) and introduced into a body fluid of a patient so that the active moiety is preferentially delivered to tumor cells by specificity of the drug-bound antibody to the tumor cell.


Brief Description of the Drawings


FIG. 1 is a schematic depiction of immunoblots of serum dilutions for samples from two immunized patients (Pt. 1, Pt. 2) and an immunized rabbit tested against the particulate fraction (lanes numbered 1) and conditioned medium (lanes numbered 2) from A375 cells. Pt. 1 received intralymphatic (I.L.) immunotherapy with intact, irradiated tumor cells, Pt. 2 received subcutaneous (S.C.) immunization, and rabbit 209 was immunized subcutaneously with the particulate fraction of A375 (ING-A) cells.

FIG. 2 is a schematic depiction of an HPLC chromatogram of a partially-purified A375-ING-A extract and the corresponding immunoblot analysis of HPLC pools; and

FIG. 3 is a schematic depiction of immunoblots in which regression associated antibodies from three different patients (A,B,C) were allowed to react with antigens fractionated from cell lysates of early (MAC-1) and late (MAC-3) passages of two different melanoma cell lines.


Detailed Description


The present invention concerns the identification of human tumor cell-associated antigens and the properties of antibodies developed against such antigens in patients responding with tumor regression following active specific immunotherapy using tumor cells or cell extracts. These novel antigens, designated herein as RAAs, have been detected using RAAbs from patient sera in a number of fresh human tumor extracts and in cultured human tumor cell lines.

Discrete antigens associated with cultured human tumor cells may be identified by screening large numbers of monoclonal antibodies produced in mice and other animals against tumor cells or partially fractionated immunogens derived therefrom. However, most antibodies directed against such antigens may not be associated with the progression or regression of tumors and as such the antigens detected by these antibodies are unlikely to have therapeutic potential as active immunogens. Furthermore, human antigenic determinant(s) which elicit an antibody response in mice or other animals might not trigger human immune surveillance mechanisms against a tumor.

In the present invention, specific antibodies have been detected in sera from patients undergoing tumor regression following active immunization by intralymphatic infusion of cells derived from their own tumors (autochthonous) or established tumor-derived cell cultures of similar histopathologic type (allogeneic). The antibodies of this invention are characterized by their specific reactivity toward certain antigens associated with certain human tumor cells. These antibodies have thus been used to identify specific antigens in tumor cells and tissues. These tumor-associated antigens, designated herein as RAAs, may be further grouped based upon their sizes, their ability to react with RAAbs from patients regressing different malignancies, and their presence and/or their relative abundance in cells obtained from different types of human cancer.

5

RAAbs according to the present invention are antibodies which are induced in response to administration of irradiated tumor cell immunogens and are associated with the stabilization or regression of tumor masses.

RAAs according to the present invention include antigens present on human tumor cells which presumably induce the production of RAAbs and which thus may be recognized by antibodies in the sera of patients showing tumor regression in response to tumor cells administered through the intralymphatic route.

The present invention also concerns methods for obtaining preparations containing such RAAs including established human tumor cell lines, protein and cell-free tissue extracts and preparations derived from membranes or culture media of such cells and tissues. Polyclonal or monoclonal antibodies against RAAs may be prepared in animals and may be used for therapeutic purposes, diagnostic tests or monitoring the course of therapy, including therapy involving active immunization protocols.

Example 1

Specific Antibodies Against Tumor Cell-Associated Antigens In Patients Showing Evaluable Tumor Regression After Immunotherapy

1(a) Serum collection. Serum samples are obtained from patients with documented malignancies that are in the state of tumor progression from the primary site of origin to other locations in the body (metastasis) or by a demonstrable growth of the primary tumor mass. The patients are then subjected to intralymphatic immunotherapy as described in Juillard et al., Bull. Cancer, 66, 217 (1979), using infusions of tumor cells obtained from their own tumors or cultured tumor-derived cells established from malignancies of the same type as the patient in question as described in Juillard et al., Cancer, 41, 2215 (1978); and in Weisenburger et al., J. Biol. Response Mod., 1, 57 (1982). The amount of neoplastic cells used for immunization and methods of their processing including washing and irradiation prior to administration into patients is presented by Juillard and his colleagues in published reports including Juillard et al., Cancer, 41, 2215-2225, (1978) and is also described in Bubbers et al., Bull. Cancer, 68, 332-337 (1981).

Neoplastic cells used in immunization are often defined as cells derived from tumors isolated from cancer patients, which cells may have karyotype different from that of cells derived from corresponding normal tissues and may have chromosomal abnormalities and which cells, when allowed to proliferate in culture, may have altered metabolic and growth properties. These altered growth properties include one or more of the following: (a) the loss of anchorage-dependent growth and the ability to form masses in semi-solid media; (b) the ability to produce solid tumors in athymic mice; and (c) alterations in membrane structures, including the presence of tumor-associated antigens, examples of which are described in the literature and which are distinct from the antigens described in this invention. See Hood et al., Immunology, Benjamin/Cummings Publishing Co., 510-529, (1984); and Sell, in Proceedings of the UCLA Symposium on Monoclonal Antibodies and Cancer Therapy, supra, 3-21 (1985).

A state of tumor regression in patients undergoing intralymphatic immunotherapy described above is established by evaluating the status of the tumor using several criteria. The evaluation process comprises accepted clinical monitoring procedures including standard radiological evaluations, computerized axial tomography, magnetic resonance imaging, assessment of various immunological markers for cancers (e.g., carcinoembryonic antigen, newly discovered antigens detected by tumor-specific monoclonal antibodies), detection of tumor-specific enzymes and hormone receptors, and other standard laboratory tests used in the clinical management of cancer patients. In addition, a careful physical examination, including palpation of tumor nodules (wherever possible), is conducted.

Partial tumor regression is indicated if the following is observed: stabilization of a tumor which was progressing prior to immunotherapy (i.e., failure to detect any objective change in tumor size for three months), or less than fifty percent decrease in tumor size and associated subjective improvement or status quo. Such stabilization of tumor growth is associated with the development of delayed hypersensitivity to immunogens in the irradiated neoplastic cells used for treatment and is assessed by subcutaneous and intradermal skin testing of the cellular preparations.

A successful tumor regression response is defined as an objectively measurable decrease (i.e., at least fifty percent) in the size of the tumor mass. Tumor mass is assessed by direct measurement, when the tumor is near the surface of the body and directly palpable, by radiological measurements and by the additional criteria cited above.

Serum samples are obtained from patients before and at different times after initiation of the immunotherapy. The status of the tumor is assessed as described above. The serum samples from patients undergoing the immunotherapy regimen and responding with tumor regression are tested along with the serum samples from each patient obtained prior to the initiation of immunotherapy and/or before the patient is in a state of regression.

1(b) Human tumor cell lines. The following human tumor cell lines were obtained from the American Type Culture Collection (ATCC), Rockville, Maryland, were propagated according to ATCC recommendations and were shown to express RAAs using the Western immunoblotting technique as described in section 1(d) below: A375 melanoma cells (ATCC No. CRL 1619); LoVo colonic adenocarcinoma cells (ATCC No. CCL229); A549 lung adenocarcinoma cells, (ATCC No. CLL185); and SW480 colonoic carcinoma cells (ATCC No. CCL228). Hereinafter, all references to A375 cells are to be understood to refer to the ING-A variant of A375 cells, cited above, which variant is deposited as ATCC No. CRL 9321, with the American Type Culture Collection 12301 Parklawn Drive, Rockville, Maryland 20852. The method of detecting RAAs in tumor cells as described in this invention has also demonstrated the presence of RAAs in various other human tumor-derived cell lines including certain cell lines which were maintained by Dr. G. Juillard, University of California at Los Angeles are designated 69-2 or WRO 82-1, CAL-27 and CAL-33 (squamous cell carcinomas), 69-3 or CAL-2 and FR084-1 (ovarian adenocarcinomas), BR081-1 and RR081-1 (renal cell carcinoma) and SR084-1 and HR084-1 (prostatic carcinoma), 100P3, CR081-6, CR081-8 (lung carcinoma), MR081-1, MR084-1 (adenocystic carcinoma), M7, M6, M14, M20, M78, CR081-5, RR081-3, ER081-1, WR081-1 (melanoma), DR081-1, WR082-1, AR081-1 (thyroid carcinoma). These cell lines were established by conventional techniques using tissue specimens obtained from the respective tumors, and several subcultures have subsequently been carried out under standard human cell culture conditions leading to their growth for 50 or more passages without observable cytologic changes. Samples of each cell culture were evaluated by microbial culturing, cytology or electron microscopy for viral or microbial contamination, and were shown to be free of identifiable microorganisms with the exception of Mycoplasma hyorhinis (as determined by Coriell Institute, Camden, New Jersey).

Mycoplasma infection of mammalian cell lines is known to result in changes in cellular metabolism and function. Van Diggelen et al., Exp. Cell Res., 106, 191 (1977); and Van Diggelen et al., Cancer Res., 37, 2680 (1977). In addition, mycoplasmas tend to be strongly adherent to the surfaces of mammalian cells and would be present in soluble and particulate extracts from washed cells. Butler et al., Infect. Immun., 42, 1136 (1983). Because of the suspected presence of M. hyorhinis in the A375 melanoma cell line (ING-A) and all other RAA-positive cell lines described in Section 1(b), the involvement of this microorganism in the production of RAAs either encoded for in the M. hyorhinis genome or through induction of expression of RAAs in the host human tumor cells is a legitimate possibility for experimental determination.

Two cell lines were established at INGENE from a 64 year old white female who presented with metastatic ovarian adenocarcinoma and involvement of inguinal lymph nodes. The first cell line, ING 69-1, was established from a one gram surgical biopsy specimen of an inguinal lymph node. The specimen was minced using sterile scissors, passed through a gauze mesh and incubated for one hour at 37°C with collagenase followed by an incubation with trypsin for an additional 30 minutes at 37°C. The tumor cell suspension was rinsed in cell culture medium, plated in RPMI 1640 medium (as above) and subcultured to generate sufficient numbers of cells for the active immunization of this patient [Weisenburger et al., J. Biol. Response Mod., 1, 57-66 (1982)]. Approximately 9 months after ING 69-1 was established, this patient underwent surgical removal of abdominal metastatic ovarian tumor masses and ING 69-11 cell cultures were established from tumor cells in ascites fluid from this patient.

1(c) Preparation of total cell or tissue extracts. Normal or neoplastic cells obtained from confluent cell cultures or freshly minced tissue biopsies were rinsed twice with phosphate buffered saline and then extracted using a mixture of ionic and non-ionic detergents according to the procedure of Sen et al., Proc. Nat'l Acad. Sci. (USA), 80, 1246-1250 (1983), or solely using a non-ionic detergent buffer which preferentially extracts proteins from cell membranes [10 mM Tris-HCl, pH 7.5, 1 mM EDTA, 2.5 mM EGTA, 10 mM NaMoO$_4$, 12 mM monothioglycerol, 10% glycerol, 40 $\mu$g/ml leupeptin (Sigma Chemical Company, St. Louis, Missouri) and 0.2% Triton X-100®]. Insoluble materials were removed by centrifugation at 10,000 X g and the supernatant solutions were retained. Aliquots of the supernatants containing 10 to 20 $\mu$g of cell protein were subjected to reducing sodium dodecyl sulfate-polyacrylamide gel electrophoresis (SDS-PAGE) essentially according to the procedure as described in Laemmli, Nature, 227, 680-685 (1970).

1(d) Immunoblotting. The detection of discrete RAAs is achieved using a Western immunoblotting technique where antibodies, RAAbs, from a patient serve as probes to detect specific antigens. Screening proteins obtained from a variety of normal and tumor cells with antibodies from a number of patients exhibiting tumor regression allows the identification of molecular characteristics of antigens which are detected by RAAbs in patients regressing tumors. It follows that these antigens elicit the production of RAAbs, the antibodies associated with tumor regression.

The immunoblotting procedure consists of the following steps as described in Towbin, et al., Proc. Nat'l Acad. Sci (USA), 76, 4350 (1979), which reference is incorporated by reference herein. In a typical Western immunoblotting procedure, total cell protein extracts or subcellular fractions are subjected to reducing sodium dodecyl sulfate-polyacrylamide gel electrophoresis (SDS-PAGE). The proteins, which may be RAAs, are transferred by electroelution onto nitrocellulose filters which are subsequently incubated with appropriate dilutions of the test serum or antibody preparation. After the incubation of the filter with antibody, which contain RAAbs, and extensive washing, the filter is incubated with $I^{125}$-labeled Protein A of Staphylococcus aureus (which specifically binds to the Fc region of antibody molecules), washed to remove unbound Protein A and exposed to X-ray film for autoradiography. Each radioactive band represents the location of a protein species which formed an immune complex with antibodies from the test sera.

1(e) Patients, treatments and antibody responses: Evaluation of independent serum samples from certain patients. Immunoblots were performed using pre-immune sera and serum samples (post-immune sera) obtained at various times after the initiation of immunotherapy and at a time when tumor regression was noticeable and post-immune sera from three different patients including the previously described patients. All patients underwent intralymphatic immunotherapy using cell lines established from their own tumors and other cultured cells established from related tumors from other patients. Antigens having molecular weights of approximately 70,000 daltons and 43,000 daltons were readily detected in protein preparations from malignant cells of ovarian origin using the post-immune sera from these three patients while no significant immunoreactivity was observed with the pre-immune sera from the same patients. The post-immune sera of these patients did not react with antigenic species in various normal human tissue or cultured cell extracts including those from normal breast and ovary.

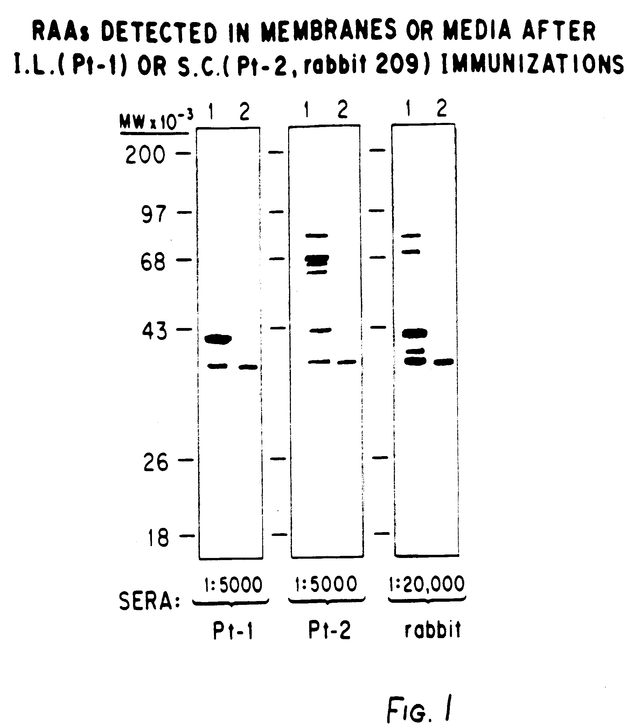

Results of immunoblot studies using post-immune sera and ascites fluid from patient A (ovarian cancer) indicated the presence of comparable antibodies in both as detected by their reactivities with a 70 kd antigen band and with two antigen bands of 38 kd to 40 kd and 43 kd to 45 kd present in several different ovarian and non-ovarian tumor cell lines. FIG. 1 presents immunoblots using 1:1000 sera dilutions from 2 immunized patients (Pt-1 and Pt-2) and a rabbit (#209) immunized with the particulate fraction obtained from A375 (ING-A) cells; all sera were tested against the particulate fraction (lanes labeled 1) or conditioned medium (lanes labeled 2) from A375-ING-A cells. The patterns of immune complex formation in this test represent what is typically observed with sera from patients regressing tumors. It was also evident from the immunoblot data that the antigen species preliminarily identified to be of about 43 kd included at least two entities with molecular weights of 38 kd to 40 kd (the "38 kd" antigen) and 43 kd to 45 kd (the "43 kd" antigen) being resolved by SDS-PAGE system of higher resolving power. Both of these antigenic species appear to be significantly different from the major histocompatibility antigens (HLA-ABC) in that their mobilities in reducing SDS-PAGE were unaffected by treatment with endoglycosidases H and F under conditions where the carbohydrate moieties of HLA-ABC is degraded by these enzymes (as visualized by immunoblots of these antigens using monoclonal antibodies specific for HLA-ABC to locate antigen bands). The 70 kd, 43 kd and 38 kd antigens are all also distinguishable from HLA-ABC in two dimensional gel electrophoresis.

A patient with metastatic prostatic cancer who received intralymphatic infusions of two irradiated cultured cells established from two independent prostatic cancers, SR084-1 and HR084-1, developed antibodies reactive with the 38 kd and the 43 kd antigens in the primary cell suspension of prostate tumors from which the HR084-1 cell line was derived; these antigens also were detected in SR084-1 cultured cells. Associated with this patient's immunotherapy were dramatic declines in serum acid phosphatase levels measured during the first two weeks following initiation of the immunizations.

The 43 kd and the 70 kd antigen species also were detected in certain fresh tumor extracts by the post-immune sera from several patients. The test extracts used in these studies were obtained from a non-Hodgkin's lymphoma and a breast carcinoma. Sera samples from a metastatic renal cancer patient were obtained before and after intralymphatic immunizations with a mixture of allogeneic renal cancer cell lines. Pre-immune serum from this patient showed negligible reactivity in the 43 kd antigen region with either of the two extracts tested; in contrast, the post-immune sera from this patient reacted with a specific 43 kd band in the lymphoma extract. In addition, a dog immunized with a human squamous cell carcinoma culture (69-2), also developed antibodies which reacted with specific antigens in the 68 kd to 70 kd range from a human Kaposi's sarcoma extract. The pre-immune sera from the patient and the dog were not reactive with the Kaposi's sarcoma extract.

Using techniques essentially as described above, antibodies in sera samples obtained from a large number of tumor-regressing patients have detected one or more of the three major molecular groups of RAAs with apparent molecular weights of about 68,000 daltons, about 43,000 daltons (also see below) and about 20,000 daltons in various tumor cells including tumor cell preparations used for immunization.

Antibodies in sera collected prior to the initiation of immunotherapy failed to detect any of these three RAAs.

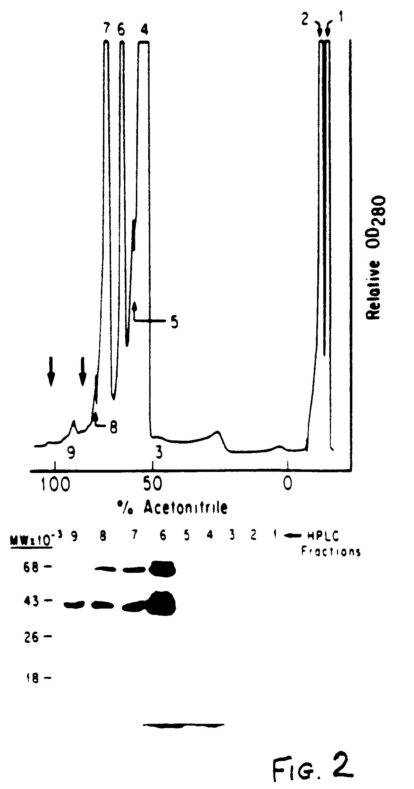

In order to further characterize these novel antigens, cell particulate fractions from human tumor cells have been used as the starting material to partially purify them. After extraction of the ING-A variant of A375 melanoma cells, ammonium sulfate fractionation and enrichment using affinity chromatography with RAAbs bound to sepharose, the 38 kd, 43 kd and 70 kd antigens were found to co-elute in an acetonitrile gradient using high performance liquid chromatography (HPLC). FIG. 2 illustrates the elution of these antigenic species as a major peak from a C-8 reverse phase HPLC column (this peak is labeled number 6 and an immunoblot analysis of antigen species in pools 1 to 9 is shown below the column profile wherein corresponding lanes are numbered to indicate correspondence to the numbered HPLC pools).

The approximate sizes of the RAAs have been estimated solely based upon the mobility, in reducing SDS-PAGE, of each of the detected RAAs relative to commercially available, pre-stained molecular weight size markers, Midrange Kit, purchased from Bethesda Research Laboratories, Inc., Bethesda, Maryland, or Diversified Biotech, Newton Centre, Massachusetts. One RAA with an assigned molecular weight of 68,000 daltons (68 kd) migrates within a size range of between 65 to 71 thousand daltons. Another RAA consists of either a single protein band of approximately 43,000 daltons (43 kd) in size within a range of between 41 to 45 thousand daltons, or frequently consists of a doublet having two detectable bands visualized on an immunoblot. The third species is an antigen of approximately 20,000 dalton molecular weight (20 kd) within a range of between 19 to 23 thousand daltons.

RAAs usually have been detected in 10 to 20 μg total protein preparations obtained from human tumor tissue and cell extracts using between 200- and 2000-fold dilutions of sera from patients regressing a variety of human malignancies including ovarian carcinoma, thyroid cancer, malignant melanoma, prostate cancer, epidermoid cancer, squamous cell carcinoma and certain others.

Table 1 summarizes results obtained with sera from five patients showing evaluable regression of the indicated metastatic cancers following intralymphatic immunotherapy. A serum sample obtained from each of the five patients (during regression) was used to perform immunoblotting studies on one or more tumor tissue extracts (designated by letter symbols) or extracts of established and primary cell cultures derived from various human tumors. The samples tested by each patient's serum sample are listed under the respective patient number. Some of the primary cell suspensions or established cultured cells have been used for intralymphatic immunotherapy and thus their abilities to induce tumor regression have been tested. These results are summarized in the second column as ILI status identified as follows: Rg +, indicating that the cell lines elicited tumor regression in ILI patients, Rg- indicating that the cell lines failed to elicit tumor regression in ILI patients, or NT indicating that the cell lines were not tested. For each of the three RAA species discussed in this table, a " + " indicates an easily detected signal in a standard immunoblot assay with the serum antibodies reacting with antigens present in 20 micrograms or less of total cellular proteins; a dilution of 200-fold or more of the sera from the indicated patient was used in all these studies. In Table 1, patient #1 had malignant melanoma, #2 had ovarian carcinoma, #3 had squamous cell carcinoma of the esophagus, #4 had prostate carcinoma and #5 had thyroid carcinoma.

## Table 1

### DISTRIBUTION OF RAAs IN TUMOR CELL LINES

| Cell line or tumor extract | Ability to Induce Regression | 43 kd RAA | 68 kd RAA | 20 kd RAA |
|---|---|---|---|---|
| PATIENT#1 | | | | |
| extract F | NT | + | + | − |
| M14 | Rg+ | + | − | − |
| 69-12 | Rg− | − | − | − |
| A375 | Rg+ | + | + | + |
| | | | | |
| PATIENT #2 | | | | |
| extract O | NT | − | + | − |
| 69-1 | Rg+ | + | + | − |
| 69-3 | Rg+ | + | + | − |
| 69-11 | Rg− | − | + | − |
| FRO81-2 | Rg+ | + | − | − |
| A375 | Rg+ | + | + | + |
| | | | | |
| PATIENT#3 | | | | |
| extract L | NT | − | + | − |
| 69-2 | Rg+ | + | + | − |
| 69-13 | Rg+ | − | + | − |
| SRO82-3 | NT | − | + | − |
| | | | | |
| PATIENT#4 | | | | |
| extract P | NT | + | + | − |
| SRO84-1 | Rg+ | + | + | − |
| | | | | |
| PATIENT #5 (cancer) | | | | |
| ARO81-1 | Rg+ | + | − | − |
| DRO82-1 | Rg+ | + | − | − |

In all of the patients presented in Table 1, immunoblotting analysis carried out with pre-immunotherapy sera failed to detect the presence of antibodies reacting with any of the respective RAAs. In several other cases evaluated, with the exception of two patients with malignant melanomas, the results of immunoblotting analysis carried out with pre-immunotherapy serum failed to reveal radioactive bands at about 68 kd, at about 43 kd or at about 20 kd, suggesting that most cancer patients prior to therapy do not contain antibodies against the RAAs of this invention. The detection of antibodies against the 38 kd and the 43 kd RAAs in the two melanoma patients prior to treatment with intralymphatic immunotherapy may be indicative of the known ability of melanomas to occasionally undergo spontaneous regression and to mediate such regression through the patients ability to generate such antibodies without active immunization.

The molecular mass determinations for the RAAs identified in Table 1 were made using "mini" SDS-PAGE in which 10% SDS-polyacrylamide resolving gels having a height of 7.5 cm are employed. When the "mini" SDS-PAGE was calibrated with molecular mass standards, the estimated principle sizes of the RAAs were of 68,000 daltons, 43,000 daltons and 20,000 daltons, although the doublet near 43,000 daltons was often not well-resolved in these gels. More precise molecular mass estimates have been obtained using the higher resolving power of a longer [14 cm] 10% SDS-polyacrylamide gel (as are employed in Fig. 1). Such gels calibrated with midrange molecular weight markers of molecular wights between 12 kd and 95 kd have indicated the molecular mass estimate to be 70,000 daltons rather than 68,000 daltons for the larger RAA and also has clearly resolved the 43,000 dalton doublet into two species: 43,000 daltons (43 kd) and 38,000 daltons (38 kd). Through the use of the higher resolving SDS-PAGE, the 43 kd RAA identified in Table 1 for patients No. 1, 2 and 4 was shown to contain large amounts of the 38 kd RAA.

In additional studies of patients subjected to immunotherapy but in whom no clinical responses were observed (i.e., in whom progression of the malignant condition continued as judged by clinical criteria including radiological analysis or direct physical examination), the results were analogous to those obtained using sera from patients prior to immunotherapy. Sera from many non-responding patients with several different types of malignancies were evaluated at different times after initiating intralymphatic immunotherapy and all of these failed to react with any of the aforementioned RAAs in extracts of tumor tissues and tumor cell lines tested.

The simple method of immunoblotting using serum samples from patients on extracts of tumor cells which contain RAAs as demonstrated by experimental results of this invention allows one to monitor the success of an immunotherapy protocol with regard to specific immune response to tumor cell-associated antigens. The monitoring may be accomplished by examining RAAb titer(s) (defined as the maximal serum dilution able to detect RAAs in a standard immunoblot procedure as described below) and RAAb specificities (defined as the apparent molecular masses of the specific antigens recognized by the serum samples).

Antibodies against tumor cell-associated components developed in patients immunized by the intralymphatic route with irradiated human tumor cell lines. These antibodies may be useful in the characterization of appropriate tumor cells or subcellular components for active specific immunotherapy of various cancers and were identified as follows.

1(f) Screening of antibody response in a large number of patients. More than fifty cancer patients were treated with a mixture of autologous human tumor cells and allogeneic human tumor cell lines by intralymphatic administration according to procedures described above. In Table 2, is a detailed presentation of patients with regard to the type of cancer, the cell lines administered, the antigens detected by their sera after immunotherapy and their clinical outcome. Cellular antigens observed in a substantial number of patients who underwent immunotherapy included membrane antigens of molecular weight 70 kd, 43 kd and 38 kd using the higher resolving SDS-PAGE for molecular weight assignment. A few other antigens with various sites of cellular localization have been noted in isolated cases and have been listed in Table 2.

In Table 2, + = remission, 0 = stable, - = progression and ? = unknown. Delayed hypersenitivity skin tests were evaluated 24 and 48 hours after intradermal injection of $10^5$ irradiated tumor cells: S = small (5-10 mm diameter of induration and erythema), M = medium (10-25 mm diameter of induration and erythema) and N = negative. Tumor designations are the following: ov = ovarian, Sq = squamous cell, ENT = head and neck, pan = pancreas, AdLu = adeno carcinoma of lung, mel = melanoma, thy = thyroid. Antigens are indicated by the number corresponding to their size in kilodaltons. Where the letter "c" follows the antigen number the antigen identified was cytoplasmic in localization (e.g., 70c). Where the letter "n" follows, the antigen number, the antigen identified was nuclear in localization (e.g., 43 n). Where the number alone is listed (e.g. 43), the antigen is localized in the tumor cell membrane. "Cell lines" are human tumor cell lines used for immunization. The cell lines are designated with letter and number codes and cell suspensions prepared from fresh antologous tumor biopsies are designated RTH alone or RTH followed by a number indicates the clinical response could not be evaluated. A small number of patients show reactivity with a 68 kd antigen in addition to reacting with the 70 kd species. This 68 kd species has not yet been directly asociated with tumor regression.

## Table 2

Summary of Clinical and Immune Response
Of Patients as Grouped by a Cell Line Which
Was Common Among Those Used for Immunization

69-2

| Patient | Tumor | Cell Lines | Antigens | Response | Skin Test |
|---|---|---|---|---|---|
| 32 | ov | 69-2 69-11T3 MCF-5 69-1 | 38, 43, 70 | 0 | |
| 21 | Sq | 69-2 CAL-33 | 38, 43, 70 | 0 | |
| 23 | Sq | 69-2 A375 | 38, 43, 70 | 0 | |
| 56 | Sq | 69-2 100P3 Cal27 RTH | 70c | - | S |
| 28 | Sq | 69-2 | | - | S |
| 14 | ENT | 69-2 CAL-27 100P3 | ? | ? | |
| 15 | ENT | 69-2 CAL-27 100P3 | 68, 70 | ? | |
| 16 | ENT | 69-2 CAL-27 100P3 | 43 | ? | |
| 17 | LUNG | 69-2 CRO81-6 100P3 CRO-81-8 | | - | |
| 18 | Gast | 69-2 A375 RTH1719 | | - | M |
| 20 | Brea | 69-2 A375 RTH964 | 38, 43, 70 | + | |
| 40 | Lung | 69-2 RTH 1717 A375 | | ? | |

EP 0 237 252 B1

| | | S | N |
|---|---|---|---|
| | | ? | I | O |

| Lung | 69-2 A375 | 46 |
|---|---|---|
| Pan | 69-2 A375 RRO81-4 | 19 |
| AdLu | 69-2 M-6 CRO81-6 | 57 |

A375

| Patient | Tumor | Cell Lines | Antigens | Response | Skin Test |
|---------|-------|------------|----------|----------|-----------|
| 22 | Sq | A375 MRO81-1 MRO84-1 | 38, 43, 70, 43c | 0 | M |
| 23 | Sq | A375 69-2 | 38, 43, 70 | 0 | |
| 1 | Mel | A375 CRO81-5 | 38, 43 | + | S |
| 2 | Mel | A375 CRO81-5 M14 WRO81-1 | 18, 41, 43, 70 | + | S |
| 29 | Thy | A375 DRO81-1 WRO82-1 RTH1722 | 38, 43, 70 | - | M |
| 10 | Mel | A375 CRO81-5 Rth | 38, 43, 70 | 0 | S |
| 18 | Gast | A375 69-2 RTH1719 | | - | M |
| 20 | Brea | A375 69-2 RTH964 | 38, 43, 70 | + | |
| 40 | Lung | A375 RTH 1717 69-2 | | ? | |
| 46 | Lung | A375 WO 69-2 | | ? | |
| 19 | Pan | A375 69-2 RRO81-4 | | - | S |
| 58 | Mel | A375 CRO81-5 RTH | | 0 | S |

EP 0 237 252 B1

EP 0 237 252 B1

$100P_3$

| Patient | Tumor | Cell Lines | Antigens | Response | Skin Test |
|---|---|---|---|---|---|
| 24 | Sq | 100P3 | 43 | + | S |
| 56 | Squ | 100P3 69-2 Cal27 RTH | 68c | − | S |
| 14 | ENT | 69-2 CAL-27 100P3 | ? | ? | |
| 15 | ENT | 69-2 CAL-27 100P3 | 68, 70 | ? | |
| 16 | ENT | 69-2 CAL 27 100P3 | 43 | ? | |
| 17 | Lung | 69-2 CRO81-6 100P3 CRO81-8 | | − | |

M-14

| Patient | Tumor | Cell Lines | Antigens | Response | Skin Test |
|---|---|---|---|---|---|
| 2 | Mel | A375 CRO81-5 M14 WRO81-1 | 18, 38, 43, 70 | + | S |
| 3 | Mel | M14 M7 M20 CRO81-5 RRO81-3 | 43c | + | N |
| 8 | Mel | M-78 M7 M14 M20 | | 0 | N |
| 47 | Mel | M14 CRO81-5 RTH-1513 | 43, 43c | − | N |
| 27 | Mel | ERO81-1 M14 | | 0 | |
| 6 | Mel | M7 M14 M20 | | − | S |

EP 0 237 252 B1

**CAL33**

| Patient | Tumor | Cell Lines | Antigens | Response | Skin Test |
|---|---|---|---|---|---|
| 21 | Sq | 69-2 CAL 33 | 38, 43, 68 | 0 | |

**CRO81-5**

| Patient | Tumor | Cell Lines | Antigens | Response | Skin Test |
|---|---|---|---|---|---|
| 1 | Mel | A375 CRO81-5 | 38, 43 | + | S |
| 2 | Mel | A375 CRO81-5 M14 WRO81-1 | 38, 43, 68, 18 | + | S |
| 3 | Mel | M14 M7 M20 CRO81-5 RRO81-3 | 43c | + | N |
| 4 | Mel | CRO81-5 M14 | | 0 | |
| 7 | Mel | M-7 M-14 CRO81-5 | | + | |
| 9 | Mel | M-7 M-14 CRO81-5 WRO82-1 | | ? | |
| 10 | Mel | A375 CRO81-5 RTH | 38, 43, 68 | 0 | S |
| 42 | Mel | CRO81-5 M-14 RTH 1746 | | 0 | S |
| 58 | Mel | A375 CRO81-5 RTH | | 0 | S |
| 47 | Mel | M14 CRO81-5 RTH 1513 | 43, 43c | - | N |

As shown in Table 2, the majority of the patients who had progression of their tumors failed to elicit an antibody response to the 38 kd, 43 kd, and 70 kd antigens present primarily in extracts of A375 melanoma cells and 69-2 cells (squamous cell carcinoma of the esophagous).

In a typical immune response after intralymphatic immunotherapy (ILI), tumor cell-associated antigens having molecular weights of approximately 70 kd and 43 kd were readily detected in malignant cells of ovarian origin using the post-immune sera from patients numbered 23 and 52 in Table 2. No significant immunoreactivity was observed with the preimmune sera from the same patients. These antigens were present in the particulate fraction of A375 cell lysates as assessed by Western immunoblots.

The majority of the patients showing evaluable response developed antibodies to particulate-associated antigens having molecular masses of 38 kd, 43 kd and 70 kd. In several cases, however, the positive clinical response could not be correlated with the appearance of these antibodies.

As shown in Table 2, eight of twelve patients treated with A375 cells along with other cells responded to the therapy. The tumors progressed in two patients, and in two others, the clinical response could not be ascertained. Of the eight positive responders, seven developed antibodies to the particulate antigens having molecular weights of about 38 kd, 43 kd, and 70 kd. None of the non-responders or the ones of questionable status developed any antibodies to the membrane antigens.

Fifteen patients were treated with 69-2 cells along with other cells. Of these, only five showed a positive clinical response, five did not respond to the therapy, and in the remaining five patients the clinical status could not be ascertained. Four of the five positive responders had developed antibodies to membrane antigens 38 kd, 43 kd, and 70 kd. The majority of the other patients did not develop antibodies to the membrane antigens.

1(g) <u>Antibody response and clinical outcome.</u> Determination of specificities and titers of RAAbs from over 50 patients undergoing intralymphatic immunotherapy have served to monitor the correlation between the development of antibodies to one or more of the RAA's of this invention and tumor regression or stabilization.

Tables 3 and 4 summarize the data which indicate an association of elevated RAAbs (anti-regressen antibodies) with clinically evaluable regression or stabilization in the patients described in Table 2.

## Table 3

RAAbs and Clinical Response in Patients
Undergoing ILI with Allogeneic and/or
Autochthonous Tumor Cells.

| RAAb Antibody Response | Number of Patients | Clinical Response | | |
|---|---|---|---|---|
| | | Regression | Stabilization | Progression |
| Strong and Broad | 10 | 4 | 6 | 0 |
| Marginal and Narrow | 15 | 3 | 3 | 9 |
| Negative | 25 | 3 | 9 | 13 |

EP 0 237 252 B1

## Table 4

RAAbs and Clinical Response in Patients Undergoing ILI with One or More of the Cell Cultures (A375-ING-A and/or 69-2 cells) Chosen For RAA Isolation.

| Antibody Response | Number of Patients | Clinical Response | | | |
|---|---|---|---|---|---|
| | | Regression | Stabilization | Progression | Unknown |
| Anti-RAA | 13 | 5 | 5 | 2 | 1 |
| No Anti-RAA | 17 | 1 | 6 | 6 | 4 |

1(h) Identification of RAAbs in melanoma patients immunized subcutaneously with melanoma cell lysates. Two cell cultures derived from melanoma tissues of two individual female patients were combined after a final passage for 4 days in serum-free medium. A mechanical lysate was prepared with a Potter-Elvejhem homogenizer at 4° C and the lysate was diluted to a known concentration of tumor cell equivalents (i.e., an amount of lysate corresponding to the number of intact cells present prior to cell lysis, generally

$10^7$ to $10^8$ cell equivalents per milliliter).

A first batch of lysate vaccine was called MAC-1 and used to treat an initial 8 patients with widespread melanoma. Based upon results of certain tests, MAC-1 cells may have been contaminated with Mycoplasma hyorhinis. Another batch, prepared in a very similar way with later passages of the cultured melanoma cells was called MAC-3 and used to treat the remaining patients in the study. MAC-3 cells do not appear to have been contaminated with Mycoplasma hyorhinis. The melanoma cell lysates were administered subcutaneously to patients along with an experimental adjuvant called DETOX™ (a detoxified fraction mycobacteria consisting principally of monophosphoryl lipid A, supplied by Ribi Immunochem Research, Inc., Hamilton, Montana). The adjuvant is known to have immune-stimulating activity and, therefore, the clinical response observed in melanoma patients may be due, at least in part, to an adjuvant effect (non-specific) in addition to the active immunization process.

Immunoblot studies were conducted using the post-immune sera from patients treated with MAC-1 (patients #1 to 8 the results are presented in Table 5) and MAC-3 (patients #9 to 15) to screen three different antigen preparation. The molecular masses in kilodaltons of the antigens detected by each patient sera are indicated under each extract. Clinical responses are indicated by : + = extensive regression, 0 = stabilization or partial regression, - = progression, and ? = not able to be evaluated.

Table 5

Immunoreactivity of Patients

| Patient No. | A375 Membranes | MAC-3 Extract | MAC-1 Extract | Clinical Response |
|---|---|---|---|---|
| 1 | 41, 43, 68, 70 | - | 41, 43, 75, 70 | + |
| 2 | 38, 41, 43, 70 | - | 38, 41, 43 | + |
| 3 | - | - | - | - |
| 4 | 43, 65, 78 | - | 60 (faint) | - |
| 5 | 38, 41, 43, 68, 70 | - | 38, 41, 43, 55 75 | + |
| 6 | 36, 38, 41, 43, 68, 70 | - | 38, 41, 43, 80 | ? |
| 7 | 43 faint | - | - | - |
| 8 | 26, 28, 38, 41, 43, 68, 70 | - | 38, 41, 43, 80 | ? |
| 9 | - | - | - | - |
| 10 | 45 (faint) | - | - | - |
| 11 | - | - | 43 (faint) | ? |
| 12-15 | -- | - | - | ? |

As indicated in the above Table 5, six out of eight patients receiving MAC-1 developed antibodies primarily to 38 kd, 43 kd and 70 kd RAAs and two of these patients showed tumor regression. An additional patient (#5) showed regression when he was subsequently treated with interleukin 2. None of the patients immunized with MAC-3 developed antibodies reactive with any of the RAAs except for faint reactivities seen by patients # 10 and #11 to single antigen bands; it should be noted that such weak reactivities were also

22

detectable with sera from these patients prior to their immunizations. The positive clinical responses seen in a few patients treated with MAC-3 may have been caused by the adjuvant since the responses were generally observed within days or weeks after immunotherapy was initiated, a time earlier than that usually required for the development of specific RAAbs.

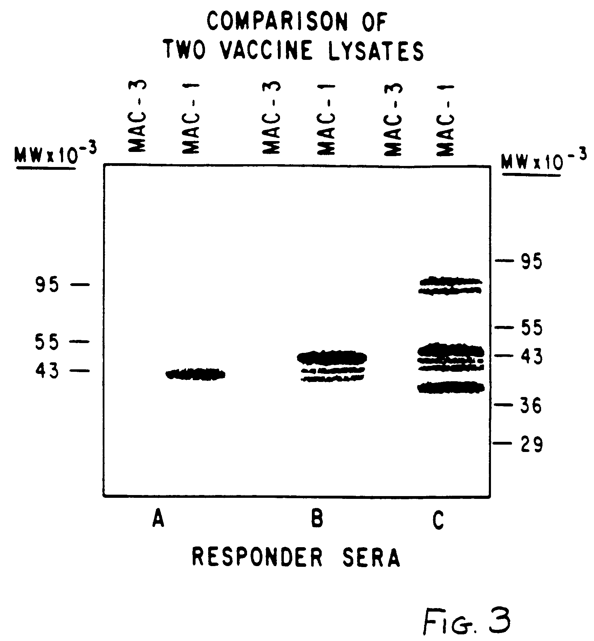

FIG. 3 illustrates the results of three immunoblots in which RAAbs from three different ILI patients (selected from responders in Table 2) were allowed to react with antigens in MAC-1 or MAC-3 extracts. The major reactivity observed with each antiserum was with 38 kd and 43 kd RAAs in MAC-1 alone. Molecular weight (MW) markers on the left were used for the Western blot with antiserum A. The two Western blots (MAC-3, MAC-1) with antisera B and C have as MW references those on the right side (near C).

These results may be compared with FIG. 1 in which the reactivity of antibodies in post-immune sera from Patient No. 2 with the 43 kd, 38 kd and 70 kd RAAs from ING-A cells is illustrated. This patient responded clinically to subcutaneous immunizations with the MAC-1 lysate preparation.

When the ING-A cell lysate is prepared according to the protocol used for preparing MAC-1 lysate, RAAs are localized in the insoluble fraction obtained as a pellet by centrifugation at 10,000 X g for 15 minutes. The soluble supernatant is free of RAAs as assessed by immunoblot analysis.

Example 2

Characterization of RAAs

2(a) Size on SDS-polyacrylamidge gel electrophoresis. The specific RAAs of this invention are distinguishable from one another and also from other antigens associated with tumors and cells derived from tumors based upon their sizes and immunological properties. Carcinoembryonic antigen (CEA) is much larger than any of the RAAs, as are several of the antigenic determinants which may be identified with existing murine monoclonal antibodies. Human chorionic gonadotropin (HCG) consists of two subunits both of which are distinguishable from the RAAs described in a reducing SDS-PAGE system (the $\beta$ subunit of HCG is 35 kd in size and its $\alpha$ subunit is 16 kd in size [Pierce et al., Ann. Rev. Biochem., 50, 465-495 (1981)]).

There is a rapidly growing list of tumor-specific antigens identified with murine monoclonal antibodies. The available information distinguishes these antigens from RAAs of this invention on the basis of size or relatedness to normal cellular constituents (Monoclonal Antibodies and Cancer Therapy, Reisfeld et al., eds., supra). Monoclonal antibodies have lead to the detection of a 92 kd, a 23 kd, and a 17 kd antigens in urinary bladder cancer, Ben-Aissa et al., Br. J. Cancer, 52, 65-72 (1985). However, these monoclonals do not react with melanoma cells (the principal source of the 19 kd to 23 kd RAA described herein).

A monoclonal antibody specific for a 43 kd surface protein of human leukemia cell line (THP-1) cross-reacts with the intermediate filament vimentin found in normal cells. Herman et al., J. Cell Sci., 73, 87-103 (1985). A 52 kd protein is released by human breast cancer cells [Capony et al., Biochem. Biophys. Res. Commun., 108, 8-15 (1982)], and a high molecular weight glycoprotein (220 kd to 400 kd) as well as a 90 kd protein are found in membranes of human breast cancer [Schlom et al., Cancer, 54, 2777-2794 (1984)]. A sarcoma-specific 70 kd antigen appears to be different from the 68 kd to 70 kd RAA of this invention in that the sarcoma 70 kd antigen was not detectable in carcinoma cell lines [Feit et al., Cancer Res., 44, 5752-5756, (1984)].

Metabolic labeling of ING-A (A375) cells with $^{35}$S-methionine. A375 cells were grown in 100 mm petri dishes to 70% confluency and were incubated with 10 ml of serum-free RPMI medium followed by fresh methionine-deficient media containing 3 $\mu$l of $^{35}$S-methionine (1148 Ci/nmol, 1 mCi/0.077 ml) (NEN Research Products, Boston, Massachusetts). Both cells and media were harvested at 1, 2, 4.5, 22 and 28 hours after exposure to $^{35}$S-methionine.

Antigens were immunoprecipitated by treating $^{35}$S-methione-labelled extracts of A375 cells with protein-A sepharose-bound IgG from a responding patient. The antigens were visualized by autoradiography following SDS-PAGE of immunoprecipitated antigens [according to the procedure of Sen et al., Proc. Nat'l Acad. Sci. (USA), 80, 1246-1250 (1983)]. Proteins immunoprecipitated from conditioned media and cell extracts by pre-immune IgG bound to protein-A Sepharose™ served as controls. Numerous bands, including a doublet around 38 kd and 43 kd molecular weight, appeared in the immunoprecipitation lanes of cell extracts with RAAbs. In contrast, immunoprecipitation of the conditioned media using RAAbs showed one band in the 38 kd region. The appearance of this band at 4.5 hours coincided with the appearance of a 38 kd protein species in the conditioned media at 4.5 hours. This experiment demonstrates that the 38 kd antigen is secreted by the A375-INGA cells.

2(b) Partial purification of RAAs from tumor cell extracts. Cell line A375 is a malignant melanoma cell line described by Todaro and colleagues. Giard et al., J. Nat. Cancer Inst., 51, 1417-1423 (1973). In a typical procedure for the isolation of RAAs, A375 cells growing in roller bottles may be washed and subsequently exposed to extraction buffers containing nonionic and ionic detergents as described above which detergents are capable of soublizing proteins from cell membranes or total cellular proteins. After high speed centrifugation (10,000 X g, 4° C) the soluble supernatant is used for further purification using conventional chromotography steps as well as affinity chromatography (e.g., resins containing immobilized antibodies reactive with regression-associated antigens or lectins with high affinity for these antigens).

Approximately $10^8$ A375 cells growing in roller bottles were rinsed twice with standard phosphate buffered saline (PBS) and then were extracted with a small volume (50 ml) of 0.2% Triton X-100® buffer for 5 min. at room temperature. The solution was removed and debris was pelleted by centrifugation (10,000 X g, 4° C). The supernatant containing RAAs was passed over an antibody affinity column containing immunoglobulins from patient #2 serum containg RAAb's purified on a Zetachrom™ membrane filter according to supplier's instructions (AMF Lab Products, Meriden, Connecticut). Ten milligrams of the immunoglobulins were coupled to an Affi-gel 10™ resin according to the supplier's instructions (Bio-Rad, Richmond, California). The unbound proteins were washed with 0.02% Triton X-100®, 25 mM Tris-HCl at pH 7.5 and 0.1 M NaCl. The column was then washed with distilled water. The bound RAAs were eluted using 1 M NaCl, 0.1% NP40™ (Sigma Chemical Co., St. Louis, Missouri), 0.5% sodium deoxycholate, 0.1% sodium dodecyl sulfate, 25 mM Tris-HCl, pH 7.5, 1% aprotinin. The eluant was dialyzed against 0.01 M ammonium bicarbonate, frozen and lyophilized.

The lyophilized powder was suspended in 0.05% trifluoracetic acid and 4 mg total protein was loaded onto a standard C-8 column for high performance liquid chromatography (Beckman Instruments). Elution was performed using a linear 0 to 100% acetonitrile gradient going from peak 1 (pass through) to peak 10. A sample from each peak was lyophilized and analyzed by immunoblotting using post-ILI serum (1:1000 dilution) from Patient #2 of Table 1. The Coomassie blue-stained SDS-PAGE revealed that greater than 40% of the detectable protein in peak 6 was in species having 43 kd and 70 kd sizes as shown in FIG. 2.

The progress of purification was monitored using the immunoblotting technique to verify the location of specific 68 to 70 kd, 38 kd and 43 kd regression-associated antigens. The degree of purity of products is assessed by determination of protein concentration as well as comparison of total protein pattern in specific fractions containing RAA's with that of the starting material. The total protein patterns of different samples were monitored by staining the SDS-PAGE with Coomassie blue protein stain or with silver staining to provide a more sensitive indication of total proteins. This general scheme has been used to successfully purify the 43 kd and 70 kd antigens from the A375 cell line and has proven effective also for comparable purification from human ovarian and squamous carcinoma cell lines. The general scheme is also expected to permit purification of the 20 kd antigen from A375 cells and of RAA's from all other cell lines and tissue extracts expressing RAA's.

Example 3

Isolation of Preparations Containing RAAs

3(a) RAAs in particulate fraction tumor cell lines. Preparation of particulate fraction from cells and cell lines. Six grams wet weight of cultured tumor cells were lysed in approximately four volumes of Buffer L [50 mM sodium phosphate (pH 7.2) containing 5 mM EDTA and 5 mM EGTA] by five strokes of homogenization in a motor driven Teflon™ homogenizer. The cell lysate was clarified at 3,000 X g for 10 minutes, the supernatant was saved and the pellet was suspended in three volumes of Buffer L by homogenization. This suspension was centrifuged again at 3,000 X g for 10 minutes. The supernatant was saved and the pellet was once more resuspended in three volumes of Buffer L and clarified by centrifugation as above. All three supernatants were pooled and were centrifuged at 100,000 X g for 1 hour.

The pelleted particulate preparation was resuspended in Buffer L supplemented with 50% sucrose by five passages in the motorized homogenizer. A ten milliliter sample of the suspension was layered on the bottom of a 40 ml VTi50 centrifuge tube, (Beckman Instruments, Irvine, California) containing a discontinuous gradient of 50%, 40%, 30% and 20% sucrose. Tubes were sealed and spun in VTi50 rotor (Beckman Instruments) for 2 hours at 45,000 rpm (with slow acceleration and no application of the brake).

Particulate material RAAs banded at approximately 40% sucrose. This band was harvested by direct aspiration, diluted five-fold with 10 mM phosphate buffer, pH 7.2 and centrifuged at 100,000 X g for one hour. Pelleted particulate material containing the antigens are resuspended by homogenization in 3 ml of 10 mM phosphate buffer, pH 7.2 and stored frozen at -20° C. In a typical preparation protocol approximately 6

24

mg of total protein was present in a final particulate material pellet, while approximately 190 mg of protein was discarded at various stages in purification starting with 6 g wet weight of cells.

3(b) RAAs from conditioned media of cultured tumor cells. Purification of secreted regression-associated antigens. In view of the localization of the 20 kd, 38 kd, 43 kd and 70 kd RAAs in the cell particulate fraction, it was of interest to assess whether any of these antigens could be recovered in serum-free media obtained from cultured tumor cells. Preliminary immunoblot experiments indicated that the serum-free "conditioned" media obtained from confluent cultures of A375 cells or 69-2 cell contain RAAs with the 38 kd antigen in abundance. In FIG. 1, the lanes numbered 2, contained the conditioned media RAA proteins from ING-A (A375) cell cultures.

Purification of the 38 kd antigen secreted by both A375 and 69-2 cells into the culture medium was performed as follows. Two liters of medium was conditioned, for 48 hours by of $3 \times 10^9$ A375 cells, centrifuged at 10,000 X g for 10 minutes, and then filtered through a 0.45 $\mu$ Nalgene® filter (Nalgene, Inc. Rochester, New York). The filtrate was concentrated to 50 ml using an Amicon YM10 filter (Amicon Corp., Danvers, Massachusetts) and dialyzed against two liters of Buffer A [10 mM $NaPO_4$ (pH 7.4)], followed by centrifugation at 10,000 X g for 20 minutes. The supernatant from the centrifugation step was loaded onto a DEAE-Sephacel® column (Pharmacia, Uppsala, Sweden) equilibrated with buffer A. The column was then washed with 75 ml of buffer A in order to remove unbound material. The 38 kd antigen did not bind to the column and was recovered in the unbound fraction which was concentrated on an Amicon YM10 filter to a volume of 10 ml.

The concentrated DEAE-purified antigen was applied to a heparin-agarose (Sigma Chemical Company, St. Louis, Missouri) column (2 ml) equilibrated with Buffer A. The column was sequentially washed with 20 ml of each of 50 mM, 100 mM, 200 mM and 500 mM NaCl in Buffer A. The 38 kd antigen eluted primarily in 200 mM NaCl wash, but smaller amounts also eluted in the 100 mM and 50 mM washes. The 200 mM eluent was concentrated on an Amicon YM10 filter to 1 ml, applied to a C8 reverse phase HPLC column and eluted with a 30%-60% linear acetonitrile gradient. The 38 kd antigen eluted at about 50% acetonitrile. Very similar elution properties for the 38 kd RAA have been obtained using a C4 reverse phase HPLC column. Analysis of the 38 kd material by reducing SDS-PAGE analysis followed by silver staining showed this 38 kd material to be ≥ 95% pure. In a typical preparation following the procedure described above, approximately 30 $\mu$g of 38 kd antigen preparation was obtained from about 300 mg of total starting protein present in 2 liters of media conditioned for 48 hours with $3 \times 10^9$ A375 cells.

Substantial purification of secreted 38 kd RAA was achieved using this procedure. The HPLC-purified antigen represents ten thousand-fold purification over the conditioned media, based on the amount of other contaminating proteins eliminated, as may be gathered from Table 7 in which the degree of purification achieved at various steps in the process is indicated. In Table 7, the term "fold purification" refers to the extent of purification based upon removal of contaminating proteins.

25

EP 0 237 252 B1

Table 6

Purification of 38 kd Antigen
From Condition Media of A375 Cells

| Step No. | | Volume (ml) | Total Protein (mg/ml) | Protein (mg) | Fold Purification (Approximate) |
|---|---|---|---|---|---|
| 1 | Conditioned media | 1500 | 0.15 | 225 | 1 |
| 2 | DEAE flow-thru concentrated | 10 | 0.19 | 1.9 | 110 |
| 3 | Heparin agaraose (200 mM salt eluent) | 1 | 0.178 | 0.178 | 1100 |
| 4 | HPLC C8 column, acetonitrile eluent | 1 | 0.020 | 0.02 | 9900 |

Example 4

Patient Monitoring by Examining Antibodies to RAAs

4(a) Monitoring titers of RAAbs in patients regressing tumors after intralymphatic immunotherapy. Serum samples from patients undergoing immunotherapy are tested as described above using immunoblotting of various human tumor cell extracts containing RAAs. Various dilutions of sera samples from each patient are evaluated for the ability to detect one or more of the specific size RAAs.

RAAbs have thus been quantitated in sera obtained at different times after initiating a therapy in a patient with metastatic ovarian cancer. Approximately six weeks after initiation of immunotherapy, one patient's serum had an RAAb titer of about 1:2000 against the 43 kd RAA and this titer reached 1:5000 three months later. Similar demonstrable elevations in titers of RAAbs to one or more RAA have been observed in patients as their tumors begin to regress subsequent to intralymphatic treatment of various other malignancies.

The assay presented here for the quantitation of RAAb titer may also be used in monitoring the effects of drugs on patients undergoing immunotherapy. This is illustrated by results obtained with a thyroid cancer patient who demonstrated tumor regression after intralymphatic immunotherapy but was subsequently treated with a high dose of prednisone for symptoms of shortness of breath. Such steroid therapy is known to suppress the immune system. Within 5 days after initiating high dose prednisone therapy, there was complete elimination of detectable RAAb titer against the 70 kd RAA as compared to a titer of 1:2000 detected previously in the same patient. Thus, the formation of RAAbs appears to follow a normal course of on active immune response to an antigen in that the RAAb titer is suppressed by immunosuppressants such as prednisone.

4(b) Monitoring the RAA specificities of RAAbs in patients undergoing intralymphatic immunotherapy. The techniques according to the present invention are useful in the assessment of response to immunotherapy not only for determining the titers of RAAbs but also for determining the different molecular species of the RAAs to which the RAAbs are directed. Different reactivities develop during the course of immunotherapy, as demonstrated by results for one patient with an initial reactivity directed against the 43 kd antigen followed by the appearance of RAAbs reacting with this 43 kd species as well as with the 38 kd and the 70 kd RAAs. The detection of RAAbs correlates in this patient with the complete regression of pulmonary metastasis of an ovarian adenocarcinoma. Similarly, in a second patient the detection of RAAbs to the 43 kd and the 70 kd RAAs coincided with an evaluable reduction in the size of a pulmonary metastasis of thyroid cancer as well as a profound improvement in the patient's sense of well-being, physical stamina and appetite.

Example 5

Development Of Antibodies Against Human RAAs in Animals

5(a) Preparation of High-Titer Polyclonal RAAbs in Rabbits. Antisera may be specifically produced by immunizing rabbits with injections of purified RAAs according to the present invention as follows. A first inoculation may contain membranes or soluble RAAs (purified from solubilized membranes or from conditioned medium) with Freund's incomplete adjuvant or with alum-adsorbed tetanus toxin as an adjuvant. Succeeding inoculations may contain the RAA and Freund's incomplete adjuvant. The animals are bled to obtain sera. Polyclonal antibodies may be isolated from the sera by conventional techniques known in the art, Handbook of Experimental Immunology, Vol. 3, Weir, ed., A 3.1 to A 4.10, Blackwell Scientific Publications (1978). Alternately, affinity columns containing purified RAAs bound to Affi-gel 10™ (Bio-Rad) may be prepared using supplier's instructions. Highly specific polyclonal antibodies may be prepared using this affinity column by conventional procedures. Affinity Chromatography, 41-44 and 92-95 Pharmacia AB, Uppsala, Sweden. High-titer (i.e., greater than 1:10,000) rabbit antisera specific for 38 kd, 43 kd and 70 kd human RAAs have been produced, as indicated in FIG. 1. with sera from rabbit 209.

5(b) Monoclonal antibodies. Monoclonal antibodies according to the present invention may be produced according to the procedure of Kohler et al., Nature, 256, 495 (1975) with the substitution of a preparation of RAA according to Example 4 for an antigen employed therein.

Basically, monoclonal antibodies are produced by injecting mice with immunizing doses of RAAs, as described above for rabbit immunization. Spleens are removed from the immunized animals, and spleen cells are fused to myeloma cells using a fusogen, such as polyethylene glycol. Hybridoma cells producing monoclonals are selected for in a selective growth medium such as the conventional HAT medium.

Monoclonal antibodies specific for RAAs may be isolated by chromatography from media in which such hybridomas have been cultured, Brown et al., J. Immunol., 131, 180-185 (1981).

Example 6

Use of Antibodies Directed Against Regression-Associated Antigens

6(a) Regression-associated antigen purification. Affinity chromatography using RAAbs bound to appropriate matrices such as Sepharose™ or Affi-gel™ can be used to generate highly-purified preparations of RAAs from human tumor cell extracts. Example 2(b) illustrates the successful isolation of RAAs from A375 cells using RAAb affinity chromatography as one of the purification steps.

6(b) RAA preparation for cancer immunotherapy. Since high titer human and rabbit RAAbs (Examples 1 and 5) react with specific antigens in fresh tumor-derived extracts, these antibodies offer a powerful means for the isolation of RAAs from a patient's own tumor, or tumor cell line. Extracts of gram quantities of the tumor or tumor cell line, where available, may be prepared in the cell lysis buffer described by Sen et al., Proc. Natl. Acad. Sci. (USA), 80, 1246-1250, (1983) and subsequently diluted prior to loading onto affinity columns of Sepharose coupled to high titer rabbit or human RAAbs (purified IgG fractions). Proteins specifically bound can be eluted from the columns by dissociative buffers and characterized by immunoblotting using known RAAbs and those preparations reacting positively with one or more of the RAAbs may be employed to actively immunize the given patient as part of a therapeutic regimen.

6(c) Drugs may be targeted to a tumor according to the present invention. An anti-cancer drug may be bound to a monoclonal antibody against an RAA (i.e., a monoclonal RAAb) of the sort described in Example 6. Such antibody-mediated drug delivery systems are reviewed in Rodwell et al., Biotechnology, 3, 889-894 (1985).

By introducing a monoclonal antibody specific for RAAs linked to an anticancer drug into a bodily fluid (blood, lymph or any other appropriate fluid such as cerebrospinal, etc.) of a patient, such a drug may be selectively delivered to tumor cells expressing an RAA for which the monoclonal RAAb is specific. It is anticipated that such binding of a tumor cell with an anticancer drug will preferentially exert an adverse effect on its survival.

Diagnostic in vivo imaging of malignancies using high titer polyclonal or monoclonal RAAbs coupled to a radioisotope (e.g. $I^{131}$, $^{90}Y$, $^{111}In$) or heavy metal (e.g., albumin-coated magnetite, $Fe_3O_4$) may be achieved using radioisotope or magnetic resonance scanning after administration to the patient of the coupled RAAb [Lauterbur, P.C., 47-57, in Accomplishments in Cancer Research 1985, Fortner et al., eds., J.B. Lippincott Co., 1986; Weinstein et al., pages 473-487, in Monoclonal Antibodies and Cancer Therapy, Reisfeld and Sell, editors, A.R. Liss, Inc., New York 1985].

Example 7

Use Of RAAs in Animal Model Systems to Examine Efficacy As Active Immunogens

Nine white male New Zealand rabbits were subcutaneously immunized in their footpads, three rabbits with membranes from 69-2 cells prepared as described in Example 3, three rabbits with membranes from A375 cells prepared as described in Example 3, and three rabbits with 150 μg per rabbit (3 rabbits) of the DEAE-Sephacel® flow through fraction of conditioned medium as described in Example 3. Five untreated rabbits of the same type served as controls. Immunized rabbits were boosted twice prior to tumor inoculation. All rabbits were then challenged with equal amounts ($5 \times 10^6$ viable cells each) of a rabbit squamous cell carcinoma. Tumors developed in the control rabbits and progressed rapidly leading to the death of the animals in 4 to 6 weeks. All immunized rabbits developed small tumors with extensive tumor necrosis, and all survived at least two months. Tumors completely regressed by 3 months in 6 of the 9 immunized rabbits (one from each group of three immunized rabbits failed to show tumor regression). Antibody responses in the immunized rabbits were detected by immunoblotting and found to be very similar to those observed in responding patients as in Examples 1 and 2, and also reacted with RAAs in fresh extracts of human melanoma and breast cancers and rabbit squamous cell carcinoma.

Example 8

Quantitation of RAAs to Monitor Tumors

A "Dot Blot" Immuno Assay for RAAs. A grid of 1.5 cm X 1.5 cm squares are drawn on 0.45 $\mu$m pore size nitrocellulose filter paper (Schleicher and Schuell, Inc., Keene, New York). The paper is rinsed in distilled water for 5 minutes and allowed to air dry. A 20 $\mu$l sample of a test extract presumed to contain an unknown amount of RAAs is mixed with an equal volume of 0.05M Tris HCl (pH7.4) 0.28 M NaCl, 1.4% Triton X-100®, and 0.2% SDS, and heated to 100°C for 5 minutes. The sample is centrifuged at 10, 000 X g and the supernatant is spotted with a micropipet within a square on the nitrocellulose filter. The filter is dried and then fixed for 15 minutes in a solvent containg 10% acetic acid and 25% isopropanol with constant agitation; this is followed by several rinses in water. The filter is then processed according to the immunoblotting technique described in Example 1, with the exception that a one hour incubation with goat anti-human antibody conjugated to horse radish peroxidase (1:2000) is substituted for the incubation with radioiodinated Staphylococcus protein A. After this one hour incubation and washing according to the immunoblot procedure, the individual squares of nitrocellulose are cut out and placed into individual wells of a multiwell plate (Costar, Cambridge, Massachusetts). Staining solution is added to each well in the amount of 0.5 ml of phosphate-buffered saline or isotonic citrate buffer (pH7) containing 0.6 mg/ml of o-phenylenediamine dihydrochloride supplemented with 1 $\mu$l of 30% hydrogen peroxide. The solution with the filter square is incubated in the dark for 30 minutes and color formation is stopped by adding 0.5 ml 4 N $H_2SO_4$ per well. Absorbance is then measured in a spectrophotometer at 490 nm. This assay provides a linear quantitation of RAAs when increasing amounts of A375 membranes are spotted onto the nitrocellulose filter paper.

Although the present invention is described in terms of a preferred embodiment, it is understood that modifications and improvements are possible. For example, other conventional immunological techniques such radioimmunoassay, immunoprecipitation and ELISA may be suitable for detection of regression-associated antigens.

It is also anticipated that RAAs according to the present invention may be further characterized, including amino acid sequencing and covalent modification thereof, if any, of any protein components and oligosaccharide structure determination of glycoprotein components, if any or lipid composition of associated lipid moieties, if any. It is further anticipated that the amino acid sequence of any polypeptides portions of RAAs may be obtained, and cDNA or a DNA sequence may be obtained therefrom and that antigens of the present invention may be produced using known, recombinant DNA techniques. Furthermore, fragments of RAAs and conjugate analogs and derivatives or fragments thereof of RAAs are also anticipated. Thus, any RAA preparation whether isolated from a cell line or directly from a tumor tissue, whether naturally produced or recombinantly derived, whether possessing the same structure as or the structure of a fragment of an RAA or an analog, derivate, or conjugate of an RAA or a fragment of an RAA disclosed herein are all intended to be encompassed within the scope of the present invention, as defined in the appended claims.

Accordingly, it is intended that the appended claims include all such equivalent variations which come within the scope of the invention as claimed.

The ING-A variant of A375 cells referred to above, was deposited on 12 February 1987 under the provisions of the Budapest Treaty with the American Type Culture Collection as ATCC No. CRL 9321.

## Claims

1. A method for detecting the presence of regression-associated antibodies comprising the steps of:
   obtaining a first sample of serum from a patient diagnosed as not being in a state of tumor regression and obtaining a second sample from the patient after diagnosis as being in a state of tumor regression;
   separately exposing a component of a neoplastic cell to first and to the second samples;
   determining the absence of immune complex formation between the component and antibodies in the first serum sample; and
   detecting the presence of an immune complex between an antibody in the second sample and the component.

2. A method for monitoring the response of a patient to immunotherapy for symptoms associated with a neoplasm comprising the step of:

determining a level of a regression-associated antibody in a sample of a body fluid of a patient.

3. A method for identifying regression associated antigens in a sample of a body fluid comprising the steps of:

exposing a component of a neoplastic cell to regression-associated antibodies; and

determining the presence of an immune complex between at least one of the antibodies and the component.

4. A method for purification of regression-associated antigens comprising the steps of:

obtaining a first sample of serum from a patient diagnosed as not being in a state of tumor regression and obtaining a second sample from a patient after diagnosis as being in a state of tumor regression;

disrupting a neoplastic cell into antigenic components of a solution;

chromatographically separating components of the solution into fractions; and

identifying fractions containing regression associated antigens by the presence of immune complex formation of components of a fraction with regression associated antibodies in the second sample.

5. A method according to claim 4 further comprising the steps of

exposing a component of a neoplastic cell to each of the samples;

determining the absence of immune complex formation between the component and antibodies in the first serum sample; and

detecting the presence of an immune complex between a regression associated antibody and the component in the second sample.

6. A purified and isolated antigen selected from the group consisting of:

regression associated antigens having a molecular weight as determined by reducing SDS polyacrylamide gel electrophoresis within the range of:

19,000 to 23,000 daltons;

38,000 to 45,000 daltons; and

65,000 to 71,000.

7. A purified and isolated antigen according to claim 6 wherein said antigen does not bind to cationic exchange resins at 50 mM $Na_3Po_4$ (pH7) or to heparin agarose resins at 200 mM $Na_3PO_4$ (pH 7).

8. A method of preparing materials for performing immunotherapy comprising the step of preparing an immunogenic amount of an antigen according to claim 6 for injection into a patient.

9. A method of preparing materials for performing immunotherapy according to claim 8, wherein the antigen is an antigen according to claim 7.

10. A method for monitoring tumor metastasis comprising the step of determining the level of a regression-associated antigen in a sample of a body fluid.

11. A monoclonal or monospecific polyclonal antibody exhibiting a specific immunoreactivity with an antigen according to claim 6.

12. A method for purifying an antibody according to claim 11 comprising the steps of:

contacting a substrate-bound antigen according to claim 6 with a solution containing an antibody according to claim 11; and

eluting the antibody from the antigen.

13. A method comprising the steps of:

binding a drug to an antibody according to claim 11; and

preparing the antibody-bound drug for introduction into a body fluid of a patient.

14. A method for purifying regression associated antigen useful in immunotherapy of cancer patients comprising the steps of:

contacting a substrate-bound antibody according to claim 11 with a solution containing an antigen according to claim 6; and

eluting the antigen from the antibody.

15. A method of preparing materials for performing immunotherapy of tumors comprising the steps of:

obtaining a first sample of serum from a patient diagnosed as not being in a state of tumor regression and obtaining a second sample from a patient after diagnosis as being in a state of tumor regression;

separately exposing a component of a neoplastic cell to the first and second samples;

determining the absence of immune complex formation between the component and antibodies in the first serum sample;

determining the presence of immune complex formation between a regression associated antibody and the component in the second sample; and

preparing the neoplastic cell, or membrane fractions thereof or regression associated antigens isolated therefrom for introduction into lymphatic or hematic fluid within a patient.

16. A method for the purification of a regression-associated antigen comprising the steps of:

conditioning medium with a cell line secreting a regression-associated antigen having a molecular weight as determined by reducing SDS polyacrylamide gel electrophoresis of about 38 kd;

concentrating protein present in the conditioned medium;

chromatographically separating fractions of the concentrated protein;

concentrating fractions exhibiting appreciable absorbance at 280 nm;

loading the concentrated fractions onto onto a $C_8$ column and eluting with a 0-80% acetonitrile gradient in 0.1% trifluoroacetic acid; and

retaining a fraction eluting at approximately 50% acetonitrile.

17. A purified and isolated regression associated antigen which is capable of forming an immune complex with a regression associated antibody.

18. An antigen according to claim 17 wherein said antigen is selected from the group consisting of:

regression associated antigens having a molecular weight as determined by reducing SDS polyacrylamide gel electrophoresis within the range of:

19,000 to 23,000 daltons;

38,000 to 45,000 daltons; and

65,000 to 71,000.

19. A purified and isolated antigen according to claim 18 wherein said antigen does not bind to cationic exchange resins at 50 mM $Na_3PO_4$ (pH7) or to heparin agarose resins at 200 mM $Na_3PO_4$ (pH 7).

20. An antigen according to claim 19 wherein said antigen is greater than or equal to 95% pure as determined by reducing SDS PAGE analysis followed by silver staining.

21. An antigen according to claim 20 wherein said antigen is encoded by DNA within Mycoplasma hyorhinis.

22. An antigen according to claim 20 wherein said antigen is encoded by DNA within a mammalian cell line associated with Mycoplasma hyorhinis.

23. An immunotherapeutic composition comprising:

a purified and isolated regression-associated antigen which is capable of forming an immune complex with a regression associated antibody; and

a pharmaceutically acceptable diluent, adjuvant or carrier.

24. An immunotherapeutic composition according to claim 23 wherein said regression-associated antigen is a regression-associated antigen according to claim 18.

25. The immunotherapeutic composition according to claim 23 wherein said regression-associated antigen is a regression-associated antigen according to claim 19.

**EP 0 237 252 B1**

**Patentansprüche**

1. Ein Verfahren zum Erkennen des Vorhandenseins von regressions-assoziierten Antikörpern, mit den folgenden Schritten:
Gewinnen einer ersten Serumprobe von einem Patienten, der als sich nicht in einem Zustand der Tumorregression befindlich diagnostiziert worden ist und Gewinnen einer zweiten Probe von dem Patienten nach einer Diagnose, daß dieser in einem Zustand der Tumorregression ist;
gesondertes Aussetzen einer Komponente einer neoplatischen Zelle der ersten und der zweiten Probe;
Bestimmen des Fehlens der Immunkomplexbildung zwischen der Komponente und den Antikörpern in der ersten Serumprobe; und
Erkennen des Vorhandenseins eines Immunkomplexes zwischen einem Antikörper zwischen der zweiten Probe und der Komponente.

2. Ein Verfahren zum Beobachten der Antwort eines Patienten auf eine Immuntherapie auf Symptome, die Neoplasma zugehörig sind mit dem folgenden Schritt:
Bestimmen des Gehalts eines regressions-assoziierten Antikörpers in einer Probe einer Körperflüssigkeit eines Patienten.

3. Ein Verfahren zum Identifizieren des regressions-assoziierten Antigens in einer Probe einer Körperflüssigkeit mit den folgenden Schritten:
Aussetzen einer Komponente einer neoplastischen Zelle unter regressions-assoziierten Antikörpern; und
Bestimmen des Vorhandenseins eines Immunkomplexes zwischen wenigstens einem der Antikörper und der Komponente.

4. Ein Verfahren zur Reinigung eines regressions-assoziierten Antigens mit den folgenden Schritten:
Gewinnen einer ersten Serumprobe eines Patienten, der als sich nicht in einem Zustand der Tumorregression befindend diagnostiziert worden ist und Gewinnen einer zweiten Probe eines Patienten, nachdem diagnostiziert worden ist, daß dieser in einem Zustand der Tumorregression ist;
Auftrennen einer neu gebildeten Zelle in Antigenkomponenten einer Lösung;
chromatographisches Trennen der Komponenten der Lösung in Fraktionen; und
Identifizieren von Fraktionen, die regressions-assoziierte Antigene beinhalten, durch das Vorhandensein einer Immunkomplexbildung von Komponenten einer Fraktion mit regressions-zugehörigen Antikörpern in der zweiten Probe.

5. Ein Verfahren nach Anspruch 4, weiter mit den folgenden Schritten:
Aussetzen einer Komponente einer neoplastischen Zelle zu jeder der Proben;
Bestimmen des Fehlens der Immunkomplexbildung zwischen der Komponente und den Antikörpern in der ersten Serumprobe; und
Erkennen des Vorhandenseins eines Immunkomplexes zwischen einem regressions-assoziierten Antikörper und der Komponente in der zweiten Probe.

6. Ein gereinigtes und isoliertes Antigen, ausgewählt aus der Gruppe bestehend aus:
regressions-assoziierten Antigenen mit einem Molikulargewicht bestimmt durch eine reduzierende SDS-Polyacrylamid-Gelelektrophorese in dem Bereich von:
   19.000 bis 23.000 Dalton;
   38.000 bis 45.000 Dalton; und
   65.000 bis 71.000.

7. Ein gereinigtes und isoliertes Antigen nach Anspruch 6, wobei das Antigen sich nicht an kationische Austauschkunstharze bei 50 mM $Na_3PO_4$ (pH7) oder an Heparinagarosekunstharze bei 200 mM $Na_3PO_4$ (pH 7) bindet.

8. Ein Verfahren zum Herstellen von Materialien zur Ausführung einer Immuntherapie mit dem Schritt des Bereitens einer immunogenen Menge eines Antikörpers nach Anspruch 6 zur Injektion in einen Patienten.

32

9. Ein Verfahren zum Herstellen von Materialien zum Ausführen einer Immuntherapie nach Anspruch 8, wobei das Antigen ein Antigen nach Anspruch 7 ist.

10. Ein Verfahren zum Beobachten einer Tumormetastase mit dem Schritt des Bestimmens des Gehaltes eines regressions-assoziierten Antigens in einer Probe einer Körperflüssigkeit.

11. Ein monoklonaler oder monospezifischer polyklonaler Antikörper, der eine bestimmte Immunreaktivität mit einem Antigen nach Anspruch 6 zeigt.

12. Ein Verfahren zum Reinigen eines Antikörpers nach Anspruch 11 mit den folgenden Schritten:
Inkontaktbringen eines substratgebundenen Antigens nach Anspruch 6 mit einer einen Antikörper nach Anspruch 11 beinhaltenden Lösung; und
Eluieren des Antikörpers von dem Antigen.

13. Ein Verfahren mit den folgenden Schritten:
Binden eines Mittels an einen Antikörper nach Anspruch 11; und
Bereiten des antikörpergebundenen Mittels zur Einbringung in eine Körperflüssigkeit eines Patienten.

14. Ein Verfahren zum Reinigen eines regressions-assoziierten Antigens, das bei einer Immuntherapie von Krebspatienten nützlich ist, mit den folgenden Schritten:
Inkontaktbringen eines substratgebundenen Antikörpers nach Anspruch 11 mit einer einen Antikörper nach Anspruch 6 beinhaltenden Lösung; und
Eluieren des Antigens von dem Antikörper.

15. Ein Verfahren zum Herstellen von Materialien zur Ausführung einer Immuntherapie von Tumoren mit den folgenden Schritten:
Gewinnen einer ersten Serumprobe von einem Patienten, der als nicht im Zustand einer Tumorregression befindlich diagnostiziert worden ist und Gewinnen einer zweiten Probe von einem Patienten nach einer Diagnose, daß dieser sich in einem Zustand einer Tumorregression befindet;
getrenntes Aussetzen einer Komponente einer neoplastischen Zelle mit ersten und der zweiten Probe;
Bestimmen des Fehlens einer Immunkomplexbildung zwischen den Komponenten und den Antikörpern in der ersten Serumprobe;
Bestimmen des Vorhandenseins einer Immunkomplexbildung zwischen einem regressions-assoziierten Antikörper und der Komponente in der zweiten Probe; und
Aufbereiten der neugebildeten Zelle oder von Membranfraktionen von dieser oder regressions-assoziierten Genen, die aus diesen isoliert worden sind zur Einführung in eine lymphatische oder blutigen Flüssigkeit in einem Patienten.

16. Ein Verfahren für die Reinigung eines regressions-assoziierten Antigens mit den folgenden Schritten:
Konditionieren eines Mediums mit einer Zellenreihe, die ein regressions-assoziiertes Antigen mit einem Molekulargewicht sekretiert, das durch eine reduzierende SDS-Polyakrylamid-Gelelektrophorese von etwa 38 kd bestimmt wird;
Konzentrieren von in dem konditionierten Medium vorhandenem Protein;
chromatographisches Trennen der Fraktionen aus dem konzentrierten Protein;
Konzentrieren der Fraktionen, die eine erhebliche Absorption bei 280 nm zeigen;
Laden der konzentrierten Fraktionen auf eine $C_8$ Säule und Eluieren mit einem 0 - 80 % Azetonitrilgradienten in 0,1 % Trifluorazetatsäure; und
Rückhalten einer Fraktion, die bei etwa 50 % Azetonitril eluiert.

17. Ein gereinigtes und isoliertes regressions-assoziiertes Antigen, das dazu in der Lage ist, einen Immunkomplex mit einem regressions-assoziierten Antikörper zu bilden.

18. Ein Antigen nach Anspruch 17, wobei das Antigen aus der folgenden Gruppe ausgewählt ist:
regressions-assoziierte Antigene mit einem Molekulargewicht bestimmt durch eine reduzierendes SDS-Polyakrylamid-Gelelektrophorese in dem Bereich von
19.000 bis 23.000 Dalton;
38.000 bis 45.000 Dalton; und
65.000 bis 71.000

**19.** Ein gereinigtes und isoliertes Antigen nach Anspruch 18, wobei das Antigen keine kationischen Austauschkunstharze bei 50 mM $Na_3PO_4$ (pH 7) oder an Heparinagarosekunstharze bei 200 mM $Na_3PO_4$ (pH 7) bindet.

**20.** Ein Antigen nach Anspruch 19, wobei das Antigen größer als oder gleich 95 % rein ist, bestimmt durch eine reduzierende SDS PAGE Analyse gefolgt durch Silberfärbung.

**21.** Ein Antigen nach Anspruch 20, wobei das Antigen durch DNA in Mycoplasma hyorhinis kodiert ist.

**22.** Ein Antigen nach Anspruch 20, wobei das Antigen durch DNA in einer Säugetierzellenzeile assoziiert mit Mycoplasma hyorhinis kodiert ist.

**23.** Eine immuntherapeutische Zusammensetzung mit:
einem gereinigten und isolierten regressions-assoziierten Antigen, das dazu in der Lage ist, einen Immunkomplex mit einem regressions-assoziierten Antikörper zu bilden; und
einem pharmazeutisch akzeptablen Verdünnungsmittel, Adjuvanz oder Träger.

**24.** Eine immuntherapeutische Zusammensetzung nach Anspruch 23, wobei das regressions-assoziierte Antigen ein regressions-assoziiertes Antigen nach Anspruch 18 ist.

**25.** Die immuntherapeutische Zusammensetzung nach Anspruch 23, wobei das regressions-assoziierte Antigen ein regression-assoziiertes Antigen nach Anspruch 19 ist.

**Revendications**

**1.** Procédé pour détecter la présence d'anticorps associés à une régression, comprenant les étapes consistant à :
- obtenir un premier échantillon de sérum à partir d'un patient dont le diagnostic indique qu'il n'est pas dans un état de régression tumorale et obtenir un second échantillon à partir du patient après qu'il a été diagnostiqué comme étant dans un état de régression tumorale ;
- exposer séparément un composant d'une cellule néoplasique au premier et au second échantillon ;
- déterminer l'absence de la formation d'un complexe immun entre le composant et les anticorps dans le premier échantillon de sérum ; et
- détecter la présence d'un complexe immun entre un anticorps dans le second échantillon et le composant.

**2.** Procédé pour contrôler la réponse d'un patient à l'immunothérapie pour des symptômes associés à un néoplasme, comprenant l'étape consistant à déterminer un taux d'un anticorps associé à une régression dans un échantillon d'un fluide corporel d'un patient.

**3.** Procédé pour identifier des antigènes associés à une régression, dans un échantillon d'un fluide corporel, comprenant les étapes consistant à :
- exposer un composant d'une cellule néoplasique à des anticorps associés à une régression ; et
- déterminer la présence d'un complexe immun entre au moins un des anticorps et le composant.

**4.** Procédé pour purifier des antigènes associés à une régression, comprenant les étapes consistant à :
- obtenir un premier échantillon de sérum à partir d'un patient dont le diagnostic indique qu'il n'est pas dans un état de régression tumorale et obtenir un second échantillon à partir d'un patient après qu'il a été diagnostiqué comme étant dans un état de régression tumorale ;
- rompre une cellule néoplasique en composants antigéniques d'une solution ;
- séparer, de façon chromatographique, les composants de la solution en fractions ; et
- identifier les fractions contenant les antigènes associés à la régression par la présence de la formation d'un complexe immun de composants d'une fraction avec des anticorps associés à la régression dans le second échantillon.

**5.** Procédé selon la revendication 4, comprenant de plus les étapes consistant à :
- exposer un composant d'une cellule néoplasique à chacun des échantillons ;

EP 0 237 252 B1

- déterminer l'absence de la formation d'un complexe immun entre le composant et les anticorps dans le premier échantillon de sérum ; et
- détecter la présence d'un complexe immun entre un anticorps associé à la régression et le composant dans le second échantillon.

**6.** Antigène purifié et isolé choisi dans le groupe constitué de :
- des antigènes associés à une régression ayant un poids moléculaire, comme déterminé par électrophorèse sur gel de polyacrylamide SDS réductrice, dans la plage de :
19 000 à 23 000 daltons ;
38 000 à 45 000 daltons ; et
65 000 à 71 000.

**7.** Antigène purifié et isolé selon la revendication 6, dans lequel ledit antigène ne se lie pas à des résines d'échange de cations à 50 mM $Na_3PO_4$ (pH7) ou à des résines d'agarose d'héparine à 200 mM $Na_3PO_4$ (pH7).

**8.** Procédé de préparation de matières pour réaliser une immunothérapie, comprenant l'étape consistant à préparer une quantité immunogénique d'un antigène selon la revendication 6 pour son injection dans un patient.

**9.** Procédé de préparation de matières pour réaliser une immunothérapie selon la revendication 8, dans lequel l'antigène est un antigène selon la revendication 7.

**10.** Procédé pour contrôler la métastase tumorale, comprenant l'étape consistant à déterminer le taux d'un antigène associé à une régression dans un échantillon d'un fluide corporel.

**11.** Anticorps monoclonal ou polyclonal monospécifique, présentant une immunoréactivité spécifique avec un antigène selon la revendication 6.

**12.** Procédé pour purifier un anticorps selon la revendication 11, comprenant les étapes consistant à :
- mettre en contact un antigène lié à un substrat selon la revendication 6 avec une solution contenant un anticorps selon la revendication 11 ; et
- éluer l'anticorps de l'antigène.

**13.** Procédé, comprenant les étapes consistant à :
- lier un médicament à un anticorps selon la revendication 11 ; et
- préparer le médicament lié à l'anticorps pour son introduction dans un fluide corporel d'un patient.

**14.** Procédé pour purifier un antigène associé à une régression, utile dans l'immunothérapie de patients cancéreux, comportant les étapes consistant à :
- mettre en contact un anticorps lié à un substrat selon la revendication 11 avec une solution contenant un antigène selon la revendication 6 ; et
- éluer l'antigène de l'anticorps.

**15.** Procédé pour préparer des matières pour réaliser une immunothérapie de tumeurs, comportant les étapes consistant à :
- obtenir un premier échantillon de sérum à partir d'un patient dont le diagnostic indique qu'il n'est pas dans un état de régression tumorale et obtenir un second échantillon à partir d'un patient après qu'il a été diagnostiqué comme étant dans un état de régression tumorale ;
- exposer, de façon séparée, un composant d'une cellule néoplasique au premier et au second échantillon ;
- déterminer l'absence de la formation d'un complexe immun entre le composant et les anticorps dans le premier échantillon de sérum ;
- déterminer la présence de la formation d'un complexe immun entre un anticorps associé à la régression et le composant dans le second échantillon ; et
- préparer la cellule néoplasique, ou des fractions de la membrane de celle-ci, ou des antigènes associés à la régression isolés de celle-ci, pour leur introduction dans un fluide lymphatique ou

35

hématique à l'intérieur d'un patient.

16. Procédé pour purifier un antigène associé à une régression, comprenant les étapes consistant à :
 - conditionner un milieu avec une lignée cellulaire sécrétant un antigène associé à une régression ayant un poids moléculaire, comme déterminé par électrophorèse sur gel de polyacrylamide SDS réductrice, d'environ 38 kd ;
 - concentrer la protéine présente dans le milieu conditionné ;
 - séparer, de façon chromatographique, des fractions de la protéine concentrée ;
 - concentrer les fractions montrant une absorbance appréciable à 280 nm ;
 - charger les fractions concentrées sur une colonne $C_8$ et éluer avec un gradient d'acétonitrile 0-80% dans de l'acide trifluoroacétique à 0,1% ; et
 - retenir une fraction éluant à approximativement 50% d'acétonitrile.

17. Antigène associé à une régression isolé et purifié, qui est capable de former un complexe immun avec un anticorps associé à une régression.

18. Antigène selon la revendication 17, dans lequel ledit antigène est choisi dans le groupe constitué de :
 - des antigènes associés à une régression ayant un poids moléculaire, comme déterminé par électrophorèse sur gel de polyacrylamide SDS réductrice, dans la plage de :
     19 000 à 23 000 daltons ;
     38 000 à 45 000 daltons ; et
     65 000 à 71 000.

19. Antigène purifié et isolé selon la revendication 18, dans lequel ledit antigène ne se lie pas à des résines d'échange de cations à 50 mM $Na_3PO_4$ (pH7) ou à des résines d'agarose d'héparine à 200 mM $Na_3PO_4$ (pH7).

20. Antigène selon la revendication 19, dans lequel ledit antigène présente une pureté supérieure ou égale à 95%, comme déterminé par analyse SDS PAGE réductrice, suivie par coloration à l'argent.

21. Antigène selon la revendication 20, dans lequel ledit antigène est codé par ADN dans Mycoplasma hyorhinis.

22. Antigène selon la revendication 20, dans lequel ledit antigène est codé par ADN dans une lignée cellulaire de mammifère associée à Mycoplasma hyorhinis.

23. Composition immunothérapeutique, comprenant :
 - un antigène associé à une régression isolé et purifié, qui est capable de former un complexe immun avec un anticorps associé à une régression ; et
 - un support, adjuvant ou diluant acceptable pharmaceutiquement.

24. Composition immunothérapeutique selon la revendication 23, dans laquelle ledit antigène associé à une régression est un antigène associé à une régression selon la revendication 18.

25. Composition immunothérapeutique selon la revendication 23, dans laquelle ledit antigène associé à une régression est un antigène associé à une régression selon la revendication 19.

RAAs DETECTED IN MEMBRANES OR MEDIA AFTER
I.L.( Pt-1) OR S.C.( Pt-2, rabbit 209) IMMUNIZATIONS

Fig. I

FIG. 2

COMPARISON OF
TWO VACCINE LYSATES

FIG. 3